(19) Europäisches Patentamt
European Patent Office
Office européen des brevets

(11) Veröffentlichungsnummer: **0 137 439**
**A2**

(12) **EUROPÄISCHE PATENTANMELDUNG**

(21) Anmeldenummer: **84111712.0**

(22) Anmeldetag: **01.10.84**

(51) Int. Cl.⁴: **C 07 D 405/14**
A 61 K 31/41

(30) Priorität: **08.10.83 DE 3336693**

(43) Veröffentlichungstag der Anmeldung:
**17.04.85 Patentblatt 85/16**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Möller, Hinrich, Dr.**
**Schumannstrasse 11**
**D-4019 Monheim(DE)**

(54) **Antitumormittel.**

(57) Beschrieben werden N-substituierte Polyglycidyl-4-aminourazolverbindungen mit mindestens 2 Glycidylresten. Die Verbindungen zeigen cytostatische Aktivität.

EP 0 137 439 A2

4000 Düsseldorf, den 4. 10. 1983
Henkelstraße 67

0137439
HENKEL KGaA
ZR-FE/Patente
Dr. Wi/Sr

P a t e n t a n m e l d u n g
D 6926 EP

"Antitumormittel"

Gegenstand der DE-OS 31 02 373 sind Arzneimittelzubereitungen mit cytostatischer Wirksamkeit, die als pharmakologischen Wirkstoff spezielle N-substituierte Polyglycidylurazolverbindungen enthalten.

Die dort beschriebenen Verbindungen tragen am Urazolring außer Glycidylgruppen nur Alkylsubstituenten. Wenngleich eine Reihe von Verbindungen mit sehr guter Wirksamkeit gegenüber unterschiedlichen Tumoren beschrieben werden, so besteht doch auf dem angesprochenen Arbeitsgebiet laufend ein Bedarf nach neuen wirksamen Substanzen.

Es ist somit Aufgabe der Erfindung, neue cytostatisch wirksame Derivate des 4-Aminourazols bereitzustellen und ein Verfahren zu ihrer Herstellung aufzuzeigen.

Gegenstand der Erfindung sind somit N-substituierte Polyglycidyl-4-aminourazolverbindungen der allgemeinen Formel

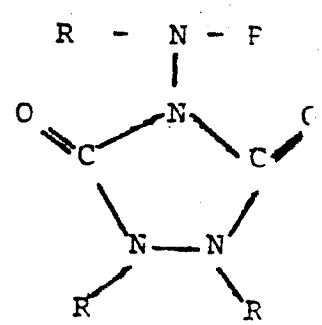

(I)

...

in der alle Reste R Glycidylreste sind oder 3 oder 2 Reste R Glycidylreste sind und die verbleibenden Reste R H oder ein C, H und gegebenenfalls Heteroatome enthaltender Rest Z sind.

Ein weiterer Gegenstand der Erfindung sind Verfahren zur Herstellung der neuen N-substituierten Polyglycidyl-4-aminourazolverbindungen sowie Arzneimittelzubereitungen mit insbesondere cytostatischer Wirksamkeit enthaltend Verbindungen der allgemeinen Formel I.

Der Wirkungsmechanismus der im Rahmen der Erfindung eingesetzten Verbindungen ist im einzelnen nicht geklärt. Vermutlich sind jedoch wie beim Triglycidylisocyanurat (DE-OS 29 07 349) die Glycidylgruppen für die cytostatische Wirkung von herausragender Bedeutung. Alle erfindungsgemäß beschriebenen Verbindungen der allgemeinen Formel I kennzeichnen sich durch das Vorliegen von wenigstens zwei solcher Glycidylgruppen. Daneben liegt gewünschtenfalls der in weiterem Rahmen variierbare Rest Z in der betroffenen Verbindungsklasse vor. Es ist möglich, daß über diesen Rest Z Einfluß auf die Verteilung von lipophilen und hydrophilen Präferenzen genommen wird und daß damit in gewissem Ausmaß die

...

Aufnahme der Verbindungen durch den Organismus oder aber speziell durch die Krebszellen gesteuert werden kann.

Der Rest Z stellt einen Wasserstoffrest oder einen Alkylrest mit bis zu 4 C-Atomen dar, der gewünschtenfalls verzweigt und/oder substituiert sein kann. So können als Reste R insbesondere Wasserstoff-, Methyl-, Ethyl-, Propyl-, Isopropyl-, Butyl- und/oder verzweigte Butylgruppen verwendet werden. Auch substituierte Reste Z sind geeignet, so zum Beispiel Ethyl-, Propyl- oder Butylgruppen, welche OH-Gruppen, Aminogruppen, Methoxy- oder Ethoxygruppen und/oder Halogenatome aufweisen.

Besonders bevorzugt sind Reste Z, die durch Ringöffnung von Glycidylgruppen mit Halogenwasserstoffsäuren oder Pseudohalogenwasserstoffsäuren entstanden sind. So sind insbesondere die durch formale Addition von Jodwasserstoff, Bromwasserstoff, Chlorwasserstoff, Fluorwasserstoff, Cyanwasserstoff oder Rodanwasserstoff entstehenden Halogeno-, Cyano- oder Rodanohydroxypropylgruppen bevorzugte Reste Z. Weitere Reste Z können durch Ringöffnung von Glycidylgruppen mit Phosphorsäure oder deren Mono- oder Dialkalimetallsalzen hergestellt werden.

Die erfindungsgemäßen Verbindungen enthalten bis zu 2 Reste Z, d. h. es sind mindestens 2 Glycidylgruppen vorhanden. Dabei können die Reste Z jede beliebige

...

Position der Reste R in der allgemeinen Formel I besetzen. So sind beispielsweise Produkte herstellbar, die beide Reste Z an der 4-Aminogruppe aufweisen oder Produkte, die die Reste Z an den beiden ringständigen Stickstoffen aufweisen. Für die cytostatische Wirksamkeit ist es nicht erforderlich, reine Isomere herzustellen, so daß auch Isomerengemische verwendet werden können.

Zur Herstellung der neuen Verbindungen ist es zweckmäßig, von 4-Aminourazol auszugehen. 4-Aminourazol ist bekannt, seine Herstellung wird zum Beispiel von L.F. Andrieth und E. Mohr in Inorganic Synth. 4, 29 (1953) beschrieben. Zur Herstellung von Verbindungen mit Alkylgruppen oder substituierten Alkylgruppen, die nicht durch Ringöffnung von Glycidylgruppen zugänglich sind, geht man von substituierten 4-Aminourazolderivaten aus, welche bereits 1 oder 2 Gruppen Z enthalten. Die Ausgangsverbindungen werden dann in die glycidylgruppenhaltigen erfindungsgemäßen Verbindungen überführt. Für diese Reaktionsstufe bestehen vor allem zwei grundsätzliche Möglichkeiten. Die eine liegt in der direkten Einführung der Glycidylgruppierung durch Umsetzung der NH-Gruppierung mit Epihalohydrin, insbesondere Epichlorhydrin oder Epibromhydrin und anschließende Dehydrohalogenierung.

...

Der andere Weg vervollständigt den Molekülaufbau in zwei Reaktionsschritten: Es werden zunächst die entsprechenden allylsubstituierten Vorprodukte gebildet, worauf in einer abschließenden Verfahrensstufe die Allylgruppe epoxidiert wird.
Zur Umsetzung von -NH-Gruppen mit Epihalohydrin besteht eine umfangreiche Literatur. Die Umsetzung kann in Gegenwart einer geringen Menge einer quartären Ammoniumverbindung als Katalysator durchgeführt werden (vergleiche hierzu beispielsweise Houben-Weyl "Methoden der organischen Chemie" Band 14/2 (1963), 497, 547).
Besonders geeignete quartäre Ammoniumverbindungen zählen zur Klasse der Phasentransfer-Katalysatoren. Hierbei handelt es sich bekanntlich um quartäre Ammoniumverbindungen mit erhöhter Lipophilie, die insbesondere durch die Gegenwart hinreichend großer organischer Reste in der quartären Ammoniumverbindung sichergestellt ist.
Ausführliche Angaben zu den Phasentransfer-Katalysatoren finden sich beispielsweise in W.P. Weber. G.W. Gobel "Phase Transfer Catalysis in Organic Synthesis", Springer-Verlag, Berlin, Heidelberg, New York, 1977 sowie E.V. Dehmlow, S.S. Dehmlow "Phase Transfer Catalysis", Verlag Chemie, Weinheim, Deerfield Beech (Florida), Basel, 1980.

...

Die Phasentransfer-Katalysatoren werden vorzugsweise in Mengen von etwa 0,1 bis 10 Gewichtsprozent, insbesondere 0,5 bis 5 Gewichtsprozent, und insbesondere 0,5 bis 3 Gewichtsprozent, bezogen auf die vorgelegte 4-Amino-urazolverbindung mitverwendet. In der nachfolgenden Reaktionsstufe wird die Dehydrohalogenierung, die teilweise auch durch überschüssiges Epihalohydrin abläuft, durch Zusatz von Basen, vorzugsweise Alkalihydroxide, vervollständigt.

Bei dem zweiten Verfahren wird die 4-Amino-urazolverbindung nicht unmittelbar mit der Epoxidverbindung umgesetzt. Statt dessen erfolgt zunächst ihre Umsetzung mit Allylhalogeniden, die dem Glycidylrest der Verbindungen der allgemeinen Formel I entsprechen, jedoch anstelle der Epoxidgruppe eine olefinische Doppelbindung aufweisen, woraufhin die entstandenen allylsubstituierten Urazole epoxidiert werden. Die Epoxidation kann in an sich bekannter Weise mit Persäuren vorgenommen werden. Als verwandte Umsetzung ist beispielsweise die Umsetzung von Cyanursäure mit Allylhalogeniden beschrieben in der US-PS 3 376 301, die Epoxidation von Allylisocyanuraten mit Persäuren ist zum Beispiel in Houben-Weyl aaO, Band 6/3, 385 ff beschrieben. Sie kann zum Beispiel in

...

Gegenwart einer geringen Menge einer quartären Ammoniumverbindung als Katalysator durchgeführt werden.

Die Umsetzung des 4-Aminourazols beziehungsweise der mono- oder di-substituierten 4-Amino-urazolverbindung mit Epi-Halohydriden beziehungsweise Allylhalogeniden erfolgt zweckmäßigerweise im Temperaturbereich von etwa 50 bis 150 °C, vorzugsweise von etwa 70 bis etwa 125 °C.

Die Reaktion kann in einem Überschuß der Epi-Halohydrinverbindung als Lösungsmittel oder in polaren aprotischen Lösungsmitteln vorgenommen werden, die wenigstens einen der Reaktionspartner teilweise lösen und den Reaktanten gegenüber nicht reaktiv sind. Ein besonders zweckmäßiges Lösungsmittel ist die Klasse der Dialkylformamide, insbesondere der niederen Dialkylformamide wie Dimethylformamid. Die bevorzugte Reaktionszeit beträgt 1 bis 20 Stunden, insbesondere 2 bis 5 Stunden.

Die Dehydrohalogenierung wird vorzugsweise mit Alkalimetallhydroxiden, insbesondere NaOH oder KOH durchgeführt. Dabei kann man sowohl mit den handelsüblichen Festprodukten als auch mit 10 - 50%igen wässrigen Lösungen arbeiten. Dabei kann es bevorzugt sein, wasserabsorbierende Mittel wie etwa Molekularsiebe zuzugeben, um das Reaktionswasser und/oder das Lösungswasser aufzunehmen. Bevorzugte Lösungsmittel für diesen Reaktionsschritt sind gegenüber Glycidylgruppen inerte Lösungsmittel wie Toluol oder Methylenchlorid. Die Reaktionstemperatur liegt zwischen 0 und 100 °C.

...

Auch die Epoxidation der Allylgruppierungen mittels Persäuren wird vorzugsweise in Lösungsmitteln durchgeführt. Geeignet sind auch hier polare Lösungsmittel, beispielsweise Halogenkohlenwasserstoffe oder Alkohole. Die geeignete Reaktionstemperatur liegt üblicherweise im Bereich von 0 bis 50 °C, insbesondere zwischen etwa 10 und 30 °C. Die Persäure wird zweckmäßigerweise in annähernd äquivalenter Menge oder nur in leichtem Überschuß eingesetzt. m-Chlorperbenzoesäure ist als Handelsprodukt leicht zugänglich und für die Durchführung der Reaktion geeignet. Die Reaktionsdauer liegt in der Regel im Bereich von 24 Stunden oder mehr, biespielsweise bis zu 48 Stunden.

Zur Herstellung von Verbindungen, die eine oder 2 Halogen-, bzw. Cyan- oder Rodan-Hydroxypropylgruppen als Substituenten Z aufweisen, ist es zweckmäßig, von 1,2-Diglycidyl-4-diglycidylamino-urazol auszugehen und diese Verbindung gezielt zum Mono- oder zum Diringöffnungsprodukt umzusetzen. Dabei wird zweckmäßigerweise nach einem von der Anmelderin in der älteren deutschen Patentanmeldung P 33 30 640.0 beschriebenen Verfahren vorgegangen. Das Verfahren der älteren Anmeldung ist dadurch gekennzeichnet, daß die mehrfunktionellen Glycidylverbindungen, also zum Beispiel 1,2-Diglycidyl-4-diglycidylaminourazol mit Halogenwasserstoffsäuren, Blausäure, Rodanwasserstoff oder Phosphorsäure oder deren Alkalimetallsalzen in wäßrigem Medium bei Temperaturen zwischen 0 und 60 °C im pH-Bereich zwischen 2 und 10 und dabei innerhalb einer Bandbreite von höchstens zwei Einheiten der

...

pH-Skala bei einem Ansatzverhältnis von 1 bis 20 mol Säure oder ihrem Alkalimetallsalz pro mol umzusetzender Epoxidgruppe zur Reaktion gebracht wird, bis die vorbestimmte Menge an Epoxidgruppen abreagiert hat. Dabei ist es bevorzugt, zur Einstellung und/oder Konstanthaltung des pH-Wertes die Säuren in dem Ausmaß dem Reaktionsgemisch zuzusetzen, wie sie durch die Reaktion verbraucht werden. Weiterhin kann nach einer speziellen Ausgestaltung des dort beschriebenen Verfahrens ein Alkalimetallsalz der Säuren eingesetzt werden und der pH-Wert mit einer vergleichsweise schwer reagierenden Fremdsäure - etwa Schwefelsäure - geregelt werden. Bevorzugt ist weiterhin das Arbeiten bei Temperaturen zwischen 20 und 40 °C sowie bei pH-Werten zwischen 3 und 8, insbesondere zwischen 4 und 7. Der molare Überschuß an Säure bzw. ihren Alkalimetallsalzen erübrigt sich bei Säuren mit reaktiven Anionen zum Beispiel bei Jodwasserstoffsäure und deren Salzen. Bei Chlorwasserstoff und dessen Salzen kann ein bis zu 20facher Überschuß eingesetzt werden.

Gegenstand der Erfindung ist schließlich weiterhin die Verwendung der Verbindungen der allgemeinen Formel I zur Therapie maligner Neoplasmen. Einzelgaben der Verbindungen in Höhe von 1 bis 200 mg/kg können zweckmäßig

...

sein. Es können einzelne, bestimmte Verbindungen der allgemeinen Formel I oder Mischungen von Ihnen zum Einsatz kommen. Auch ihre Verwendung in Abmischung mit anderen Wirkstoffkomponenten, zum Beispiel Triglycidylurazol, fällt in den Rahmen der Erfindung.

Die erfindungsgemäß eingesetzten Verbindungen der allgemeinen Formel I treten in verschiedenen stereoisomeren Formen auf. Grundsätzlich eignen sich alle diese verschiedenen Formen für die Zwecke der Erfindung. Sie können dabei in Mischung oder auch in Form bestimmter isolierter Isomeren eingesetzt werden.

Zur Verwendung als Cancerostatica sollten die Wirksubstanzen mittels geeigneter Vehikel appliziert werden. Hier eignen sich die üblichen Hilfs- beziehungsweise Trägerstoffe für pharmakologische Zubereitungen. In vorliegendem Fall hat sich die Verwendung von wäßrigen Sytemen, gegebenenfalls zusammen mit verträglichen Glykolethern wie Glykolmonoethylether oder Butylenglykolmethylether oder Propylenglykolmethylether bewährt, insbesondere wenn der Wirkstoff parenteral appliziert werden soll. Bei oraler Verabreichung sind die pharmazeutisch üblichen Hilfs- beziehungsweise Trägerstoffe anwendbar, sofern sie eine entsprechende

...

Verträglichkeit mit den Glycidylverbindungen aufweisen.

Im Tierexperiment hat sich die Verwendung von frisch hergestellten, wäßrigen Lösungen, die i.p. oder i.V. gegeben werden, als zweckmäßig erwiesen.

Die erfindungsgemäß eingesetzten Verbindungen sind wirksam gegen verschiedene Leukämieformen sowie maligne Neoplasmen wie Lungencarcinom, Coloncarcinom, Melanome, Ependymoblastom und Sarcome. Es hat sich gezeigt, daß in einigen Fällen eine deutliche Überlegenheit gegenüber Cyclophosphamid und Fluoruracil festzustellen ist.

Eine Kombinationstherapie in Verbindung mit anderen Cytostatica wie Derivaten des Stickstofflosts oder auch Fluoruracil ist möglich.

Ganz allgemein gilt für die im Rahmen der Erfindung eingesetzten Verbindungen der allgemeinen Formel I mit einem Rest Z, daß dieser Rest Z wenigstens unter Normalbedingungen oder wenigstens unter Kühlung keine oder keine wesentliche Reaktivität mit den Epoxidgruppen des beziehungsweise der Glycidylsubstituenten am Ringsystem der allgemeinen Formel I zeigen, beziehungsweise zeigen soll.

...

Auf diese Weise ist sichergestellt, daß die erfindungsgemäß verwendeten Wirkstoffe hinreichend lagerbeständig sind und keine unerwünschte Umsetzung unter Vernichtung der Epoxidgruppierungen stattfindet. Diese Regel ist insbesondere auch bei der Auswahl eventuell vorliegender Substituenten an dem Rest R zu berücksichtigen.

Die erfindungsgemäß beschriebenen Polyglycidyl-substituierten 4-Amino-urazole liegen in den Arzneimittelgemischen gemäß der Erfindung üblicherweise in Konzentrationen bis zu 20 Gewichtsprozent - bezogen auf die Arzneimittelmischung - vor. Geeignet ist beispielsweise der Bereich von 0,05 bis 10 Gewichtsprozent, insbesondere ein Bereich von 0,05 bis 5 Gewichtsprozent.

Die Prozentangaben der nachfolgenden Beispiele beziehen sich jeweils auf Gewichtsprozent, soweit nicht im Einzelfall andere Angaben gemacht sind.

...

B e i s p i e l

1,2-Diglycidyl-4-diglycidylamino-urazol

Die Mischung aus 14,4 g (0,125 mol) 4-Amino-urazol, 0,25 g Tetramethylammoniumchlorid, 0,2 g Benzyl-dodecyldimethyl-ammoniumchlorid und 276 g (3 mol) Epichlorhydrin wurde 6 Std. zum Sieden erhitzt, das überschüssige Epichlorhydrin unter vermindertem Druck abdestilliert, der Rückstand in 150 ml Methylenchlorid aufgenommen, die Lösung mit 5,88 g Natriumhydroxid-Pulver 1,5 Std. gerührt und nach Filtrieren eingeengt. Der Rückstand (8,7 g) wurde in wenig Methylenchlorid aufgenommen und an einer Kieselgelsäule (Fa. Merck) chromatographiert. Elutionsmittel: Methylenchlorid/Essigester/Methanol (3:2:1), 10 Fraktionen à 100 ml. Die Fraktionen 4 und 5 enthielten das Endprodukt (2,4 g) schwach verunreinigt. Nach erneuter Chromatographie wurden 1,9 g reines 1,2-Diglycidyl-4-diglycidylamino-urazol vom Berechnungsindex $n_D^{20}$: 1,4725 erhalten.

Epoxid-O: gef. 18,94 %; ber. 18,82 %

...

Die nachfolgenden Versuche wurden durchgeführt nach Testvorschriften des National Cancer Institute Bethesda, Maryland 200014, veröffentlicht in "Cancer Chemotherapy Reports" Part. 3, September 1972, Vol. 3, Nr. 2. Als Wirksubstanz wurde 1,2-Diglycidyl-4-diglycidylamino-urazol des vorstehenden Beispiels 1 verwendet. Die Substanz wurde als wäßrige, 1%ige Injektionslösung unmittelbar vor der Applikation frisch hergestellt.

Bei Mäusen wurde gemäß Protokoll 1200 (S. 91c) die Tumorart P 388 (Leukämie) i.p. mit 10 Zellen/Maus gesetzt. Die mittlere Überlebensdauer der unbehandelten Tiere wird bestimmt.

In weiteren Versuchsgruppen wird entsprechend vorbehandelten Tieren der Wirkstoff appliziert. Es wird eine signifikante Verlängerung der Lebensdauer der behandelten Testtiere gegenüber der mittleren Überlebensdauer der nicht mit dem Wirkstoff behandelten Tiere erzielt. Die Verlängerungsrate T/C in Abhängigkeit von der Dosierung des Wirkstoffes ist in der folgenden Tabelle zusammengestellt:

...

Tabelle

| Substanz | verabreichte Dosis mg/kg | T/C-Wert | 30 Tage überlebende Tiere (von 6) |
|---|---|---|---|
| 1,2-Diglycidyl-4-diglycidyl-aminourazol | 50 | >300 | 6 |
| | 25 | 155 | |
| | 12,5 | 142 | |
| | 6,25 | 291 | 3 |
| Triglycidylurazol | 50 | 300 | 6 |
| | 25 | 260 | 4 |
| | 12,5 | 230 | |
| | 6,25 | 180 | |

## Patentansprüche

1. N-substituierte Polyglycidyl-4-aminourazolverbindungen der allgemeinen Formel

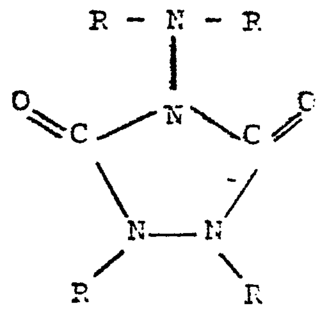

(I)

in der wenigstens 2 der Reste R den Glycidylrest bedeuten und die verbleibenden Reste R gegebenenfalls Heteroatome aufweisende andere Kohlenwasserstoffreste Z sind.

2. Polyglycidyl-4-aminourazolverbindungen nach Anspruch 1, dadurch gekennzeichnet, daß 1 oder 2 der Substituenten R Kohlenwasserstoffreste Z mit nicht mehr als 4 C-Atomen sind, die durch Ringöffnung von Glycidylgruppen mit Jodwasserstoff, Bromwasserstoff, Chlorwasserstoff, Blausäure oder Phosphorsäure oder ihren Mono- oder Dihydrogensalzen oder Rodanwasserstoff hergestellt worden sind.

3. Verfahren zur Herstellung von N-substituierten Polyglycidyl-4-aminourazolverbindungen nach Anspruch 1, dadurch gekennzeichnet, daß 4-Aminourazol gewünschtenfalls substituiert mit einem oder zwei Resten Z, in an sich bekannter Weise mit einem Epihalogenhydrin,

...

vorzugsweise Epichlorhydrin umgesetzt und das Zwischenprodukt mit Alkalihydroxiden dehydrohalogeniert wird.

4. Verfahren zur Herstellung von N-substituierten Polyglycidyl-4-aminourazolen nach Anspruch 1, dadurch gekennzeichnet, daß 4-Aminourazol, welches gewünschtenfalls mit 1 oder 2 Reste Z substituiert ist, in an sich bekannter Weise mit einem Allylhalogenid, insbesondere mit Allylchlorid umgesetzt wird, woraufhin die Allylreste mit organischen oder anorganischen Peroxiden zu Glycidylgruppen oxidiert werden.

5. Verfahren zur Herstellung von Polyglycidyl-4-aminourazolverbindungen nach Ansprüchen 1 und 2, dadurch gekennzeichnet, daß in einem ersten Verfahrensschritt Tetraglycidyl-4-aminourazol nach dem Verfahren der Ansprüche 3 oder 4 hergestellt wird, woraufhin in wäßrigem Medium bei Temperaturen zwischen 0 und 60 °C im pH-Bereich zwischen 2 und 10 und dabei innerhalb einer Bandbreite von höchstens zwei Einheiten der pH-Skala bei einem Ansatzverhältnis von 1 - 20 mol Säure und/oder ihrem Alkalimetallsalz pro mol umzusetzender Epoxidgruppe die Tetraglycidylverbindung mit Halogenwasserstoffsäuren oder Pseudohalogenwasserstoffsäuren oder Phosphorsäure und/oder ihren partiellen Alkalimetallsalzen zur Reaktion gebracht wird, bis die vorbestimmte Menge an Epoxidgruppen abreagiert hat.

6. Verwendung der neuen Polyglycidyl-4-aminourazolverbindungen bei der Behandlung maligner Tumore.